# EUROPEAN PATENT APPLICATION

(11) **EP 4 410 180 A2**
(43) Date of publication of application: **07.08.2024**
(21) Application number: 24154678.7
(22) Date of filing: 30.01.2024
(51) Int. Cl.: A61B 3/00, A61B 3/113, A61B 3/12

(54) **SINGLE PIXEL THREE-DIMENSIONAL RETINAL IMAGING**

(30) Priority: 02.02.2023 US 202363442958 P; 23.01.2024 US 202418420734
(71) Applicant: Meta Platforms, Inc., Menlo Park, CA 94025 (US)
(72) Inventor: An, Yatong, Menlo Park (US); Wang, Youmin, Menlo Park (US)
(74) Representative: Murgitroyd & Company

(57) **Abstract**

Systems and methods for eye tracking are disclosed. The system may emit, by a digital micro-mirror device(s) (DMD), light, including binary images or grayscale images, onto an eye(s) of a user. The system may determine, by single pixel detectors, a reflection of the light from the eye(s). The system may determine, by a first single pixel detector, a first signal associated with the reflection of the light from a retina of the eye(s). The system may determine, by a second single pixel detector, a second signal associated with the reflection of light from the retina of the eye(s). The system may perform, by the DMD(s), a transformation on the first and second signals to determine a grayscale image(s) of the retina. The system may determine, by the DMD(s), a 3D shape(s) of the retina based on performing a 3D reconstruction(s) associated with pixels of the grayscale image(s) of the retina.

## Description

### TECHNICAL FIELD

Examples of this disclosure relate generally to methods, apparatuses and computer program products for eye tracking, and specifically relate to compressive sensing for eye trackers comprising a plurality of single pixel detectors and a digital micro-mirror device as a spatial light modulator.

### BACKGROUND

Artificial reality is a form of reality that has been adjusted in some manner before presentation to a user, which may include, e.g., a virtual reality (VR), an augmented reality (AR), a mixed reality (MR), a hybrid reality, or some combination and/or derivatives thereof. AR, VR, MR, and hybrid reality devices often provide content through visual means, such as through a headset, e.g., glasses.

Many artificial reality devices utilize cameras to present information, render additive information and/or content on top of the physical world, and execute various AR operations and simulations. For example, an artificial reality device may display a hologram overlaid on top of a screen or display.

Eye tracking refers to the process of detecting the direction of a user's gaze, which may comprise detecting the angular orientation of the eye in three-dimensional space. Eye tracking may further comprise detecting the location of the eye (e.g., the center of the eye), the torsion (i.e., the roll of the eye about the pupillary axis) of the eye, the shape of the eye, the current focal distance of the eye, the dilation of the pupil, other features of the eye's state, or some combination thereof.

Eye tracking is an important feature for various systems used in artificial reality applications, e.g., head-mounted display (HMD) systems. Conventional eye tracking systems track features of the human eye and are typically limited by the quality of the optical path. These conventional systems traditionally use optical coherence tomography (OCT) devices for eye tracking. However, due to space constraints associated with HMD systems (e.g., AR glasses) and a relatively large size of conventional OCT devices, it is difficult to integrate conventional OCT devices into HMD systems. Moreover, conventional OCT devices may be prohibitively expensive to include in low-cost products intended to be affordable for consumers.

Accordingly, improvements are needed for eye tracking and processing techniques to address the increasing size and/or cost requirements for artificial reality applications.

### SUMMARY

In meeting the described challenges, the present disclosure provides systems, methods, devices, and computer program products for eye tracking using compressive sensing for eye trackers comprising a plurality of single pixel detectors and a digital micro-mirror device (DMD) as a spatial light modulator (SLM). For example, two or more single pixel detectors and a DMD device may be used to perform a three-dimensional (3D) reconstruction of the retina. Various aspects and examples may be usable on a range of artificial reality devices and applications, including but not limited to wearable devices, head-mounted systems, headsets, glasses, helmets, visors, gaming devices, and smart devices.

A DMD device may render content (e.g., binary images or grayscale images) to a user's pupil or retina. Each of a plurality of single pixel detectors may capture a signal (e.g., light) of the rendered content on the user's retina. For example, by inverse Fourier transform, each pixel detector may recover a grayscale image of a user's retina. A 3D shape of the retina may be built by using stereo 3D reconstruction techniques among the recovered pixels. Moreover, a movement or characteristic of the eye may be determined based on the recovered image or 3D shape.

According to a first aspect, there is provided an apparatus comprising: one or more processors; at least one memory storing instructions: at least one digital micro-mirror device configured to: emit light, comprising one or more binary images or one or more grayscale images, onto at least one eye of a user; a plurality of single pixel detectors configured to: determine a reflection of the light from the at least one eye; determine, by a first single pixel detector of the plurality of single pixel detectors, a first signal associated with the reflection of the light from a retina of the at least one eye; determine, by a second single pixel detector of the plurality of single pixel detectors, a second signal associated with the reflection of the light from the retina of the at least one eye; and wherein when the one or more processors execute the instructions, the apparatus is configured to: perform a transformation on the first signal and the second signal to determine at least one grayscale image of the retina; and determine at least one three-dimensional (3D) shape of the retina based on performing at least one 3D reconstruction associated with one or more pixels of the grayscale image of the retina.

When the one or more processors further execute the instructions, the apparatus may be configured to: track one or more movements of the at least one eye based in part on the determined at least one 3D shape of the retina.

When the one or more processors execute the instructions, the apparatus may be configured to: perform the track the one or more movements of the at least one eye based in part on determining one or more eye tracking parameters associated with the 3D shape of the retina.

The one or more eye tracking parameters may comprise at least one of an angle of eye-gaze of the at least one eye, a torsion of the at least one eye, a shape of the at least one eye, a current focal distance of the at least one eye, a dilation of a pupil of the at least one eye, an inter-pupillary distance of the at least one eye and a second eye of the user, a translation of the at least one eye, an accommodation of the at least one eye, other features of a state of the at least one eye, or a combination thereof.

Prior to performing the transformation, the at least one digital micro-mirror device may be further configured to: receive the first signal and the second signal from the plurality of single pixel detectors.

The at least one digital micro-mirror device may be further configured to: render content to the at least one eye based on a determined location of a pupil of the at least one eye.

When the one or more processors execute the instructions, the apparatus may be further configured to: perform the determined location of the pupil.

The apparatus may further comprise a spatial light modulator. The light may be spatially modulated, by the at least one digital micro-mirror device or by the spatial light modulator, based on the determined location of the pupil.

When the one or more processors execute the instructions, the apparatus may be configured to: determine at least one characteristic of the at least one eye based on the at least one grayscale image of the retina.

According to a second aspect, there is provided a method comprising: emitting by at least one digital micro-mirror device, light, comprising one or more binary images or one or more grayscale images, onto at least one eye of a user; determining, by a plurality of single pixel detectors, a reflection of the light from the at least one eye; determining, by a first single pixel detector of the plurality of single pixel detectors, a first signal associated with the reflection of the light from a retina of the at least one eye; determining, by a second single pixel detector of the plurality of single pixel detectors, a second signal associated with the reflection of the light from the retina of the at least one eye; performing, by the at least one digital micro-mirror device, a transformation on the first signal and the second signal to determine at least one grayscale image of the retina; and determining, by the at least one digital micro-mirror device, at least one three-dimensional (3D) shape of the retina based on performing at least one 3D reconstruction associated with one or more pixels of the grayscale image of the retina.

The method may further comprise: tracking one or more movements of the at least one eye based in part on the determined at least one 3D shape of the retina.

The method may further comprise: performing the tracking the one or more movements of the at least one eye based in part on determining one or more eye tracking parameters associated with the 3D shape of the retina.

The one or more eye tracking parameters may comprise at least one of an angle of eye-gaze of the at least one eye, a torsion of the at least one eye, a shape of the at least one eye, a current focal distance of the at least one eye, a dilation of a pupil of the at least one eye, an inter-pupillary distance of the at least one eye and a second eye of the user, a translation of the at least one eye, an accommodation of the at least one eye, other features of a state of the at least one eye, or a combination thereof.

Prior to the performing the transformation, the method may further comprise: receiving, by the least one digital micro-mirror device, the first signal and the second signal from the plurality of single pixel detectors.

The method may further comprise: rendering, by the at least one digital micro-mirror device, content to the at least one eye based on a determined location of a pupil of the at least one eye.

The method may further comprise: performing, by at least one of the plurality of single pixel detectors, the determined location of the pupil.

The method may further comprise: spatially modulating the light, by the at least one digital micro-mirror device or by a spatial light modulator, based on the determined location of the pupil.

The method may further comprise: determining at least one characteristic of the at least one eye based on the at least one grayscale image of the retina.

According to a third aspect, there is provided a computer-readable storage medium storing computer executable instructions that, when executed by a computer, cause the computer to carry out the method of the second aspect.

According to a fourth aspect, there is provided a computer program product comprising instructions which, when the program is executed by a computer, cause the computer to carry out the method of the second aspect..

Additional advantages will be set forth in part in the description which follows or may be learned by practice. The advantages will be realized and attained by means of the elements and combinations particularly pointed out in the appended claims. It is to be understood that both the foregoing general description and the following detailed description are exemplary and explanatory only and are not restrictive, as claimed.

### BRIEF DESCRIPTION OF THE DRAWINGS

The summary, as well as the following detailed description, is further understood when read in conjunction with the appended drawings. For the purpose of illustrating the disclosed subject matter, there are shown in the drawings examples of the disclosed subject matter; however, the disclosed subject matter is not limited to the specific methods, compositions, and devices disclosed. In addition, the drawings are not necessarily drawn to scale. In the drawings:
FIG. 1 illustrates an artificial reality system comprising a headset, in accordance with various examples of the present disclosure.
FIG. 2 illustrates a block diagram of a retinal imaging system, in accordance with examples of the present disclosure.
FIG. 3 illustrates a flowchart for eye tracking, in accordance with various examples of the present disclosure.
FIG. 4 illustrates another flowchart to facilitate eye tracking, in accordance with another example of the present disclosure.
FIG. 5 illustrates a block diagram of an example device in accordance with various examples of the present disclosure.
FIG. 6 illustrates a block diagram of an example computing system in accordance with various examples of the present disclosure.
FIG. 7 illustrates a machine learning and training model in accordance with various examples of the present disclosure.
FIG. 8 illustrates another computing system in accordance with various examples of the present disclosure.

The figures depict various examples for the purpose of illustration only. One skilled in the art will readily recognize from the following discussion that alternative examples of the structures and methods illustrated herein may be employed without departing from the principles described herein.

### DETAILED DESCRIPTION

The present disclosure can be understood more readily by reference to the following detailed description taken in connection with the accompanying figures and examples, which form a part of this disclosure. It is to be understood that this disclosure is not limited to the specific devices, methods, applications, conditions, or parameters described and/or shown herein, and that the terminology used herein is for the purpose of describing particular examples by way of example only and is not intended to be limiting of the claimed subject matter.

Some examples of the present invention will now be described more fully hereinafter, with reference to the accompanying drawings, in which some, but not all examples of the invention are shown. Indeed, various examples of the invention may be embodied in many different forms and should not be construed as limited to the examples set forth herein. Like reference numerals refer to like elements throughout. As used herein, the terms "data," "content," "information," and similar terms may be used interchangeably to refer to data capable of being transmitted, received, and/or stored in accordance with examples of the invention. Moreover, the term "exemplary", as used herein, is not provided to convey any qualitative assessment, but instead merely to convey an illustration of an example. Thus, use of any such terms should not be taken to limit the scope of examples of the invention.

As defined herein a "computer-readable storage medium," which refers to a non-transitory, physical, or tangible storage medium (e.g., volatile or non-volatile memory device), may be differentiated from a "computer-readable transmission medium," which refers to an electromagnetic signal.

As referred to herein, a Metaverse may denote an immersive virtual space or world in which devices may be utilized in a network in which there may, but need not, be one or more social connections among users in the network or with an environment in the virtual space or world. A Metaverse or Metaverse network may be associated with three-dimensional virtual worlds, online games (e.g., video games), one or more content items such as, for example, images, videos, non-fungible tokens (NFTs) and in which the content items may, for example, be purchased with digital currencies (e.g., cryptocurrencies) and/or other suitable currencies. In some examples, a Metaverse or Metaverse network may enable the generation and provision of immersive virtual spaces in which remote users can socialize, collaborate, learn, shop and engage in various other activities within the virtual spaces, including through the use of Augmented/Virtual/Mixed Reality.

As referred to herein, an interpupillary distance (IPD) may be a determined distance between the eyes of a user. In some examples, the IPD may be a distance between the centers of both pupils of the eyes of the user.

References in this description to "an example", "one example", or the like, may mean that the particular feature, function, or characteristic being described is included in at least one example of the disclosed subject matter. Occurrences of such phrases in this specification do not necessarily all refer to the same example, nor are they necessarily mutually exclusive.

Also, as used in the specification including the appended claims, the singular forms "a," "an," and "the" include the plural, and reference to a particular numerical value includes at least that particular value, unless the context clearly dictates otherwise. The term "plurality", as used herein, means more than one. When a range of values is expressed, another example includes from the one particular value and/or to the other particular value. Similarly, when values are expressed as approximations, by use of the antecedent "about," it will be understood that the particular value forms another example. All ranges are inclusive and combinable. It is to be understood that the terminology used herein is for the purpose of describing particular aspects only, and is not intended to be limiting.

It is to be appreciated that certain features of the disclosed subject matter which are, for clarity, described herein in the context of separate examples, can also be provided in combination in a single example. Conversely, various features of the disclosed subject matter that are, for brevity, described in the context of a single example, can also be provided separately, or in any sub-combination. Further, any reference to values stated in ranges includes each and every value within that range. Any documents cited herein are incorporated herein by reference in their entireties for any and all purposes.

In various aspects, systems, and methods enable eye tracking (e.g., including 3D retinal imaging) and processing techniques utilizing a DMD as an SLM and one or more single pixel detectors. For example, 3D retinal imaging is important for eye tracking applications, such as foveated rendering. Traditional retinal imaging systems are usually based on OCT technologies. However, due to the size of OCT, it may be hard to integrate OCT into an HMD or AR glasses. For example, it may be desirable for AR glasses to have a similar streamlined profile as that of a traditional glasses frame. By utilizing compressive sensing technologies, aspects may greatly reduce the size requirements of HMDs or AR glasses.

Moreover, different types of sensors may perform in different ways for eye tracking. For example, cameras, event sensors, fringe sensors, range tracking sensors, indirect time of flight (iTOF), time of flight (TOF) sensors, or photosensors (e.g., for photosensor oculography (PSOG)) may be utilized to track eye motion. It is difficult for conventional sensors to achieve certain operational requirements and features, such as low power consumption, high speed, high accuracy, high precision, small size, low latency, and field of view (FOV) considerations. Such operational requirements and features are becoming increasingly important as applications, such as headsets, AR/VR devices, or glasses become more advanced.

Accordingly, aspects of the disclosed subject matter may utilize a 3D retinal imaging system based on compressive sensing technologies. In various aspects, the imaging system may include two or more single pixel detectors (e.g., photodiode (PD), avalanche photodiode (APD), single-photon avalanche diode (SPAD)). The imaging system may include a DMD or SLM device to do 3D reconstruction of the retina. For example, a DMD device may project binary images or grayscale images onto the retina and the single pixel detectors may capture the signal. By inverse Fourier transform, each single pixel detector may recover a grayscale image of the retina. By using stereo 3D reconstruction technologies among grayscale images, a 3D shape of the retina may be built. Hence, the packaging and cost advantages of the single pixel detectors may be realized.

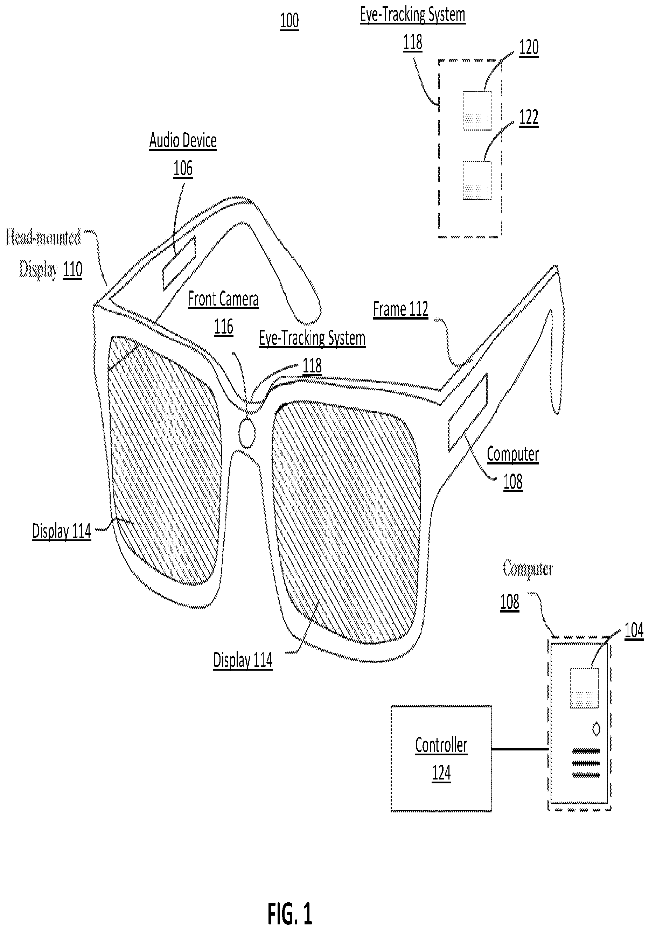

FIG. 1 illustrates an example artificial reality system 100. In some examples, the artificial reality system 100 may be utilized in a Metaverse network. In other examples, the artificial reality system 100 may be utilized in any suitable network capable of provisioning content and/or facilitating communications among entities within, or associated with the network. The artificial reality system 100 can include a head-mounted display (HMD) 110 (e.g., glasses) comprising a frame 112, one or more displays 114, and a computing device 108 (also referred to herein as computer 108). The displays 114 can be transparent or translucent, allowing a user wearing the HMD 110 to look through the displays 114 to see the real world and displaying visual artificial reality content to the user at the same time. The HMD 110 can include an audio device 106 (e.g., speaker/microphone) that can provide audio artificial reality content to users. The HMD 110 can include one or more cameras 116 which can capture images and videos of environments.

The HMD 110 can include an eye tracking system 118 to track the vergence movement of the user wearing the HMD 110. In some aspects, the eye tracking system 118 may include one or more single pixel detectors 120 (e.g., PD, APD, SPAD). Moreover, the eye tracking system 118 may include one or more DMDs 122 as an SLM. The eye tracking system 118 may determine a 3D reconstruction of a retina of a wearer of the HMD 110. For example, the DMD(s) 122 may project binary images or grayscale images onto the retina and the single pixel detectors 120 may capture the signal. By inverse Fourier transform, each single pixel detector 120 may recover a grayscale image of the retina. By using stereo 3D reconstruction technologies among grayscale images, a 3D shape of the retina may be built by the eye tracking system 118.

The eye tracking system 118 may determine a movement or a characteristic of an eye of the user based on the recovered grayscale image or the 3D shape of the retina. Moreover, the eye tracking system 118 may estimate a position and angular orientation of the eye of a user wearing the HMD 110. The position and angular orientation of the eye corresponds to the direction of the user's gaze within the HMD 110. The eye tracking system 118 may also detect the torsion of the eye, i.e., rotation of the eye about the pupillary axis. The eye tracking system 118 may also track a change in the shape of the eye, which may be approximated as a skew or scaling linear transform or a twisting distortion (e.g., due to torsional deformation).

As the orientation and position may be determined for both eyes of the user, the eye tracking system 118 is able to determine where the user is looking. The HMD 110 can use the orientation and position of the eye to, e.g., determine an inter-pupillary distance (IPD) associated with eyes of the user (e.g., a distance between the eyes of the user), determine gaze direction, introduce depth cues (e.g., blur image outside of the user's main line of sight), collect heuristics on the user interaction in the artificial reality media (e.g., time spent on any particular subject, object, or frame as a function of exposed stimuli), some other function that is based in part on the orientation of at least one of the user's eyes, or some combination thereof. Determining a direction of a user's gaze may include determining a point of convergence based on the determined orientations of the user's left and right eyes. A point of convergence may be the point that the two foveal axes of the user's eyes intersect (or the nearest point between the two axes). The direction of the user's gaze may be the direction of a line through the point of convergence and though the point halfway between the pupils of the user's eyes.

The eye tracking system 118 can be positioned between the user's eye and the optical assembly. In alternate examples, the eye tracking system 118 may be placed between the optical assembly and the electronic display or within the optical assembly. The eye tracking system 118 may include an illumination source (e.g., DMD 122), an imaging device (e.g., one or more single pixel detectors 120), and a controller 207 coupled to the imaging device or integrated into the imaging device. The illumination source (e.g., DMD 122) emits light onto a portion of the user's eye. The imaging device (e.g., one or more single pixel detectors 120) captures light reflected from the portion of the eye illuminated by the illumination source (e.g., DMD 122). The controller may be configured to determine eye tracking information for the user's eye. The eye tracking information determined by the eye tracking system 118 may comprise information about a position and orientation of the user's eye in an eye-box, such as information about a location of a pupil of the eye and information about an angle of eye-gaze. An eye-box represents a three-dimensional (3D) volume at an output of the HMD 110 in which the user's eye is located to receive image light.

In some examples, the components of the eye tracking system 118 are positioned outside an optical axis of the frame 112, e.g., the illumination source (e.g., DMD 122) and the imaging device (e.g., one or more single pixel detectors 120) are positioned outside of a primary optical path of an electronic display (e.g., a display(s) 114), irrespective of a transmitted or reflected primary optical path of the one or more displays 114. Instead, the illumination source (e.g., DMD 122) and the imaging device (e.g., one or more single pixel detectors 120) are coupled through one or more non-primary direct or reflected optical paths to the user's eye. The one or more non-primary optical paths may encompass at least part of the primary optical path of the one or more displays 114.

Based on the determined and tracked position and orientation of the user's eye, the HMD 110 may adjust presentation of one or more images displayed on the one or more displays 114. In one example, the HMD 110 may adjust resolution of the displayed image(s) based on the eye tracking information. A maximum pixel density for displaying the image(s) on the electronic display (e.g., a display(s) 114) can be provided only in a foveal region of the determined eye-gaze, whereas a lower resolution display is employed in other regions, without negatively affecting the user's visual experience. In another example, the HMD 110 may adjust focus of the displayed image(s) such that the displayed image(s) are in focus at the determined eye-gaze location, which also mitigates vergence-accommodation conflict (VAC) of image light propagating towards the user's eye. The HMD 110 may perform various other applications based at least in part on the determined eye tracking information. The applications include, but are not limited to, providing user interfaces (e.g., gaze-based selection), attention estimation (e.g., for user safety), different gaze-contingent display modes (e.g., synthetic depth of field rendering), metric scaling for depth and parallax correction (e.g., IPD and eye-box adjustment), etc.

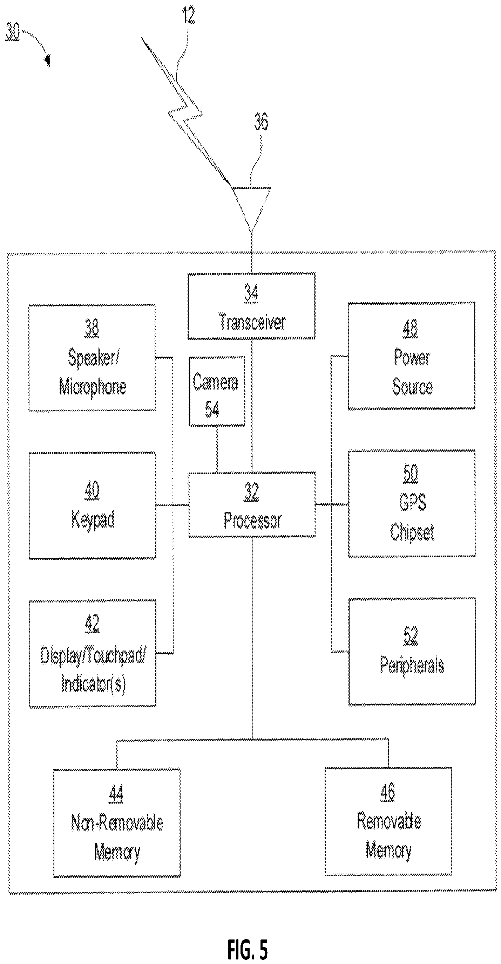

The HMD 110 can include a microphone of the audio device 106 to capture voice input from the user. The HMD 110 may include a controller 104 configured to process one or more functions/operations of the computing device 108. The artificial reality system 100 can further include a controller 124 (e.g., processor 32 of FIG. 5) and may comprise a trackpad and one or more buttons. The controller 124 can receive inputs from users and relay the inputs to the computing device 108. The controller 124 can also provide haptic feedback to users. The computing device 108 can be connected to the HMD 110 and the controller 124 through cables or wireless connections. The computing device 108 can control the HMD 110 and the controller 124, and/or controller 104, to provide the augmented reality content and/or virtual reality content to and receive inputs from one or more users. In some example examples, the controller 124, and/or controller 104, can be a standalone controller or integrated within the HMD 110. The computing device 108 can be a standalone host computer device, an on-board computer device integrated with the HMD 110, a mobile device, or any other hardware platform capable of providing artificial reality content to and receiving inputs from users. In some examples, HMD 110 can include an artificial reality system/virtual reality system (e.g., artificial reality system).

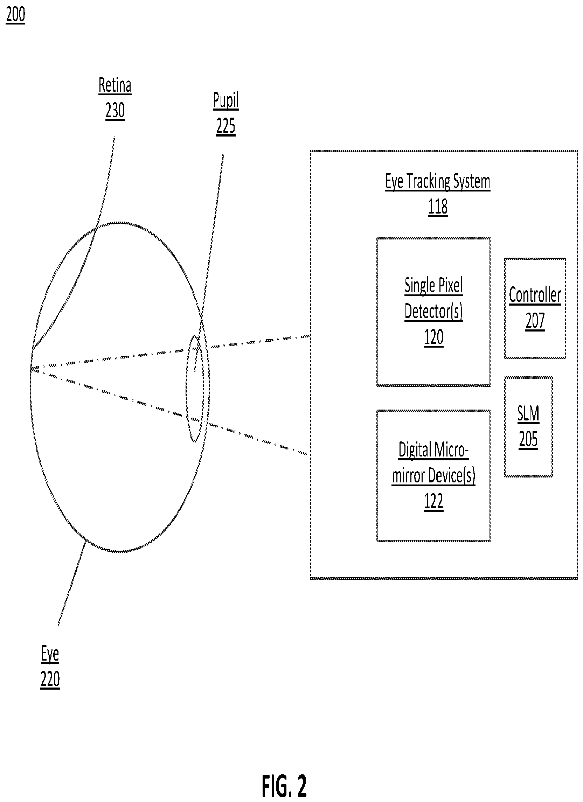

FIG. 2 illustrates a block diagram 200 of an eye tracking system 118. The eye tracking system 118 may include a DMD(s) 122 and a plurality of single pixel detectors 120. The DMD 122 may be coupled to the eye tracking system 118 such that the DMD 122 can illuminate surfaces of a user's eye 220.

The eye tracking system 118 may instruct an illumination source (e.g., DMD 122) to emit light toward the eye 220, based in part on emission instructions. In particular, the eye tracking system 118 may instruct DMD 122 to project binary images or grayscale images through the pupil 225 and onto the retina 230 of the eye 220.

The eye tracking system 118 may instruct the single pixel detectors 120 to capture a signal of the reflected light on the pupil 225 or retina 230 based on the light, and/or binary/grayscale images, emitted by the DMD 122. For example, each single pixel detector (SPD) of the single pixel detectors 120 may capture a signal (e.g., light) of content rendered onto the retina 230 by DMD 122. By inverse Fourier transform, each SPD of the single pixel detectors 120 may recover a grayscale image of the retina 230, based on the signal of content rendered onto the retina 230. By utilizing the recovered grayscale image of the retina 230, a 3D shape of the retina 230 may be built/generated, by the DMD 122, by using stereo 3D reconstruction techniques among the recovered pixels. The recovered pixels may be associated with the recovered grayscale image(s) of the retina 230. In some examples, a controller (e.g., controller 207) may perform the determining of the recovered grayscale image(s) of the retina 230. For example, for each pixel individually, among the pixels of a recovered grayscale image, a controller (e.g., controller 207) may project multiple patterns with different spatial frequencies and orientations onto the retina (e.g., retina 230). The signal obtained by the controller based on that pixel under a certain frequency and orientation may fill up a corresponding position on a two-dimensional (2D) Fourier transform map of a retina image. Then, by inverse Fourier transform of the filled map, the controller may obtain a grayscale image of the retina. With multiple pixels, the controller may obtain multiple grayscale images of the retina. Then by applying deep learning, or other computer vision techniques, the controller may predict the 3D geometry of the retina from those grayscale images.

In some other examples of the present disclosure, other approaches to determine the 3D geometry of a retina from one or more grayscale images may be utilized. For example, an eye tracking system (e.g., eye tracking system 118) may leverage deep learning techniques to predict the 3D geometry of the retina. For instance, an eye tracking system (e.g., eye tracking system 118) may feed raw information of a grayscale image(s) determined/obtained from a single pixel detector (of the single pixel detectors 120) to a neural network to enable the neural network to predict the 3D geometry of the retina (e.g., retina 230). Additionally or alternatively, an eye tracking system may feed some post-processed information (e.g., a grayscale image(s) obtained/determined by/through inverse Fourier transform) to a neural network to enable the neural network to predict the 3D geometry of the retina. In some other examples of the present disclosure, an eye tracking system may combine the Fourier transform method and deep learning method together to generate a hybrid method to determine a retinal 3D reconstruction of the retina.

Based on the captured images and/or the generated 3D shapes, the eye tracking system 118 may determine one or more eye tracking parameters. For example, the one or more eye tracking parameters may include, but are not limited to, an angle of eye-gaze, a torsion of the eye 220, a shape of the eye 220, a current focal distance of the eye 220, a dilation of the pupil 225, an IPD associated with one or more eyes of a user, a translation of the eye 220, an accommodation of the eye 220, other features of the eye's state, or some combination thereof. The eye tracking system 118 may utilize one or more of the eye tracking parameters to track one or more eyes (e.g., eye 220) of a user (e.g., a user wearing an HMD 110).

In some aspects, a spatial light modulator 205 (also referred to herein as SLM 205) may be coupled with the eye tracking system 118 to spatially modulate light. The light, that may be modulated, may be illuminated onto the eye 220 by the DMD 122. Moreover, the light may be spatially modulated, by the SLM 205, based on information about an estimated location of the pupil 225. Thus, based on the information about the estimated location of the pupil 225 determined by a controller (e.g., controller 207) based on reflected light information associated with the pupil obtained from one or more of the single pixel detectors 120, the DMD 122 may render content to the pupil 225 and further to the retina 230 of the eye 220. In some examples, the content rendered by the DMD 122 to the pupil 225 and to the retina 230 may be visual content. In some examples, the visual content may include, but is not limited to, one or more videos, pictures, animations, other multimedia content and/or the like. Additionally, in some other examples, the DMD 122 may comprise the SLM 205.

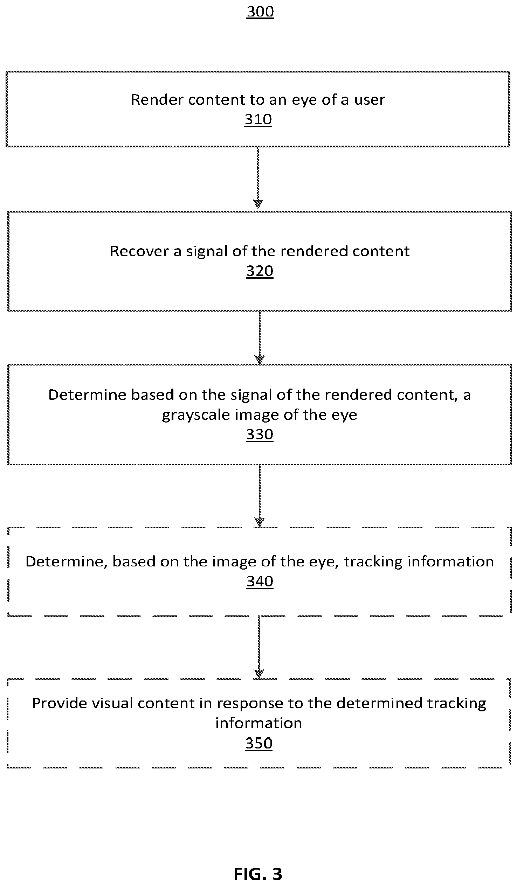

FIG. 3 illustrates a flowchart 300 for eye tracking, in accordance with various aspects discussed herein. At block 310, content may be rendered to an eye (e.g., eye 220) of a user. For example, the content may be rendered by a spatial light modulator comprising a digital micro-mirror device (e.g., DMD 122). The content may comprise binary images and/or grayscale images. Moreover, the content may be rendered to a pupil (e.g., pupil 225) of the user and/or to a retina (e.g., retina 230) of the user.

At block 320, a signal of the rendered content may be recovered from the eye. For example, the rendered content may be recovered by each of a plurality of single pixel detectors (e.g., single pixel detectors 120).

At block 330, a grayscale image of the eye may be determined based on the signal of the rendered content. For example, the grayscale image may be recovered by applying inverse Fourier transform to a recovered signal from each of the single pixel detectors.

Optionally, at block 340, the grayscale image, associated with the eye, may be used to determine tracking information associated with the eye. For example, tracking information may include, but is not limited to, eye movements, eye movement over a period of time, eye position over a period of time, and/or the like. In various examples, determining tracking information may include determining at least one of a convergence pattern or a divergence pattern based on tracked movement of the first eye and tracked movement of the second eye.

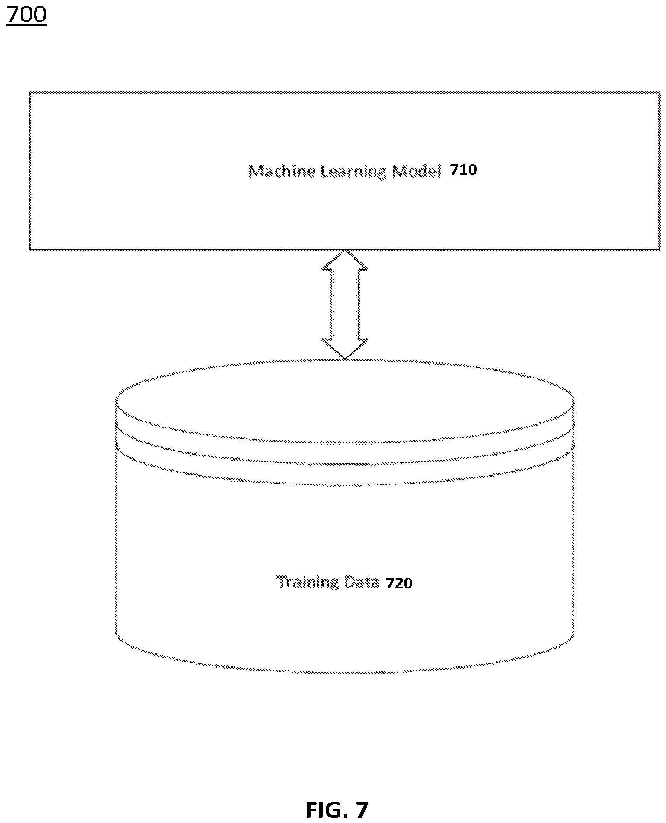

Various aspects, examples, and techniques discussed herein may utilize one or more machine learning models (e.g., machine learning model 710 of FIG. 7), such as neural networks, deep learning models, and other techniques such as object recognition, and/or the like, to assist in one or more of determining images, combining images, eye tracking determinations, e.g., based on data from the first eye and second eye, eye tracking determinations, and/or the like. Such machine learning models (e.g., machine learning model 710) may be trained on data sets associating tracked eye movements and/or gaze motion patterns, determining eye movements, via reflected light, phase shifts, visual information, and/or the like.

Optionally, at block 350, visual content may be provided in response to the determined tracking information. As discussed herein, such techniques may be implemented on systems, methods, and devices (e.g., artificial reality system 100 of FIG. 1), for example, those associated with wearable technology, such as a head-mounted device, headset, smart glasses, helmet, visor, gaming device, or other smart device. Other examples may include non-wearable applications, which may include one or more cameras and sensors positioned a distance away from a user and configured to track eye movements. For example, conferencing systems and/or video systems may utilize any or all of the techniques discussed herein to provide accurate actions and/or interactions that may rely on where a user is looking and/or other gaze patterns.

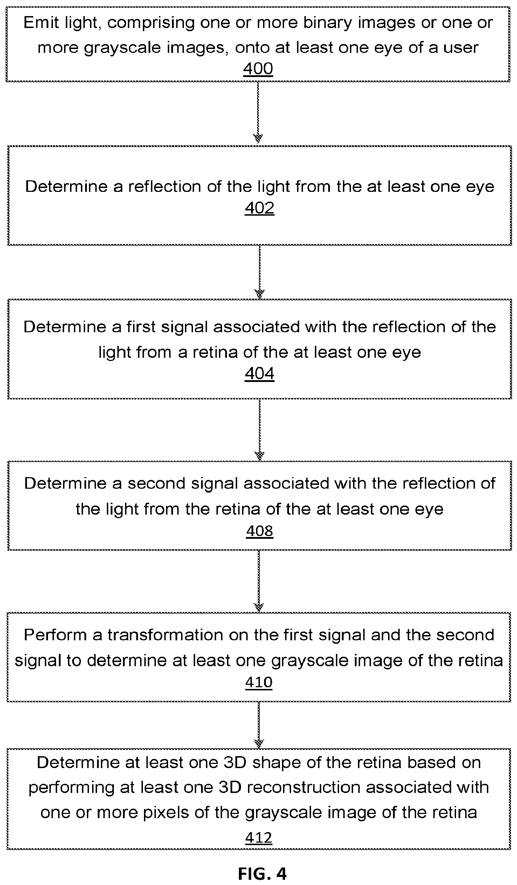

FIG. 4 illustrates an example flowchart illustrating operations to facilitate eye tracking according to another example of the present disclosure. At operation 400, a device (e.g., HMD 110) may emit light, comprising one or more binary images or one or more grayscale images, onto at least one eye of a user. In some examples, the emitting of the light may be by at least one digital micro-mirror device (e.g., one or more DMDs 122) of the device.

At operation 402, a device (e.g., HMD 110) may determine a reflection of the light from the at least one eye. In some examples of the present disclosure, the determining of the reflection may be by a plurality of single pixel detectors (e.g., single pixel detectors 120) of the device.

At operation 404, a device (e.g., HMD 110) may determine a first signal associated with the reflection of the light from a retina (e.g., retina 230) of the at least one eye (e.g., eye 220). In some examples, the determining of the first signal may be by a first single pixel detector of the plurality of single pixel detectors (e.g., single pixel detectors 120) of the device.

At operation 406, a device (e.g., HMD 110) may determine a second signal associated with the reflection of the light from the retina of the at least one eye. In some examples, the determining of the second signal may be by a second single pixel detector of the plurality of single pixel detectors of the device (e.g., single pixel detectors 120). In some other examples, determining other signals associated with the reflection of the light from the retina of the at least one eye may be by one or more other single pixel detectors of the plurality of single pixel detectors (e.g., single pixel detectors 120).

At operation 408, a device (e.g., HMD 110) may perform a transformation on the first signal and the second signal to determine at least one grayscale image of the retina. In some examples, the performing of the transformation on, or associated with, the first signal and the second signal may be by at least one digital micro-mirror device (e.g., one or more DMDs 122) of the device. In some examples of the present disclosure, the transformation may be an inverse Fourier transform on, or associated with, the first signal and the second signal to determine the at least one grayscale image of the retina. In some examples, the device may determine at least one characteristic of the at least one eye based on the at least one grayscale image of the retina.

At operation 410, a device (e.g., HMD 110) may determine at least one 3D shape of the retina based on performing at least one 3D reconstruction associated with one or more pixels of the grayscale image of the retina. In some examples, determining the at least one 3D shape of the retina may be by the at least one digital micro-mirror device (e.g., one or more DMDs 122) of the device. In some other examples, the controller 207 of the eye tracking system 118 may facilitate the operations 400, 402, 404, 406, 408 and 410 of FIG. 4. Additionally, in some other examples, the controller 207 of the eye tracking system 118 may facilitate blocks 310, 320, 330, 340 and 350 associated with the flowchart 300 of FIG. 3. In some other examples, the controller 207 may execute computer program code (e.g., computer instructions) to perform the operations 400, 402, 404, 406, 408 and 410 of FIG. 4 and/or the aspects associated with blocks 310, 320, 330, 340 and 350 associated with the flowchart 300 of FIG. 3.

FIG. 5 illustrates a block diagram of an exemplary hardware/software architecture of a device 30. As shown in FIG. 5, the device 30 can include a processor 32, non-removable memory 44, removable memory 46, a speaker/microphone 38, a keypad 40, a display, touchpad, and/or indicators 42, a power source 48, a global positioning system (GPS) chipset 50, and other peripherals 52. The device 30 can also include a camera 54. In an example, the camera 54 is a smart camera configured to sense images appearing within one or more bounding boxes. The device 30 can also include communication circuitry, such as a transceiver 34 and a transmit/receive element 36. It will be appreciated the device 30 can include any sub-combination of the foregoing elements while remaining consistent with an example.

The processor 32 can be a special purpose processor, a digital signal processor (DSP), a plurality of microprocessors, one or more microprocessors in association with a DSP core, a controller, a microcontroller, Application Specific Integrated Circuits (ASICs), Field Programmable Gate Array (FPGAs) circuits, any other type of integrated circuit (IC), a state machine, and the like. In general, the processor 32 can execute computer-executable instructions stored in the memory (e.g., non-removable memory 44 and/or memory 46) of the device 30 in order to perform the various required functions of the device. For example, the processor 32 can perform signal coding, data processing, power control, input/output processing, and/or any other functionality that enables the device 30 to operate in a wireless or wired environment. The processor 32 can run application-layer programs (e.g., browsers) and/or radio access-layer (RAN) programs and/or other communications programs. The processor 32 can also perform security operations such as authentication, security key agreement, and/or cryptographic operations, such as at the access-layer and/or application layer, for example.

The processor 32 is coupled to its communication circuitry (e.g., transceiver 34 and transmit/receive element 36). The processor 32, through the execution of computer executable instructions, can control the communication circuitry in order to cause the device 30 to communicate with other devices via the network to which it is connected.

The transmit/receive element 36 can be configured to transmit signals to, or receive signals from, other devices or networking equipment. For example, in an example, the transmit/receive element 36 can be an antenna configured to transmit and/or receive radio frequency (RF) signals. The transmit/receive element 36 can support various networks and air interfaces, such as wireless local area network (WLAN), wireless personal area network (WPAN), cellular, and the like. In yet another example, the transmit/receive element 36 can be configured to transmit and receive both RF and light signals. It will be appreciated that the transmit/receive element 36 can be configured to transmit and/or receive any combination of wireless or wired signals.

The transceiver 34 can be configured to modulate the signals that are to be transmitted by the transmit/receive element 36 and to demodulate the signals that are received by the transmit/receive element 36. As noted above, the device 30 can have multi-mode capabilities. Thus, the transceiver 34 can include multiple transceivers for enabling the device 30 to communicate via multiple radio access technologies (RATs), such as universal terrestrial radio access (UTRA) and Institute of Electrical and Electronics Engineers (IEEE 802.11), for example.

The processor 32 can access information from, and store data in, any type of suitable memory, such as the non-removable memory 44 and/or the removable memory 46. For example, the processor 32 can store session context in its memory, as described above. The non-removable memory 44 can include RAM, ROM, a hard disk, or any other type of memory storage device. The removable memory 46 can include a subscriber identity module (SIM) card, a memory stick, a secure digital (SD) memory card, and the like. In other examples, the processor 32 can access information from, and store data in, memory that is not physically located on the device 30, such as on a server or a home computer.

The processor 32 can receive power from the power source 48, and can be configured to distribute and/or control the power to the other components in the device 30. The power source 48 can be any suitable device for powering the device 30. For example, the power source 48 can include one or more dry cell batteries (e.g., nickel-cadmium (NiCd), nickel-zinc (NiZn), nickel metal hydride (NiMH), lithium-ion (Li-ion), etc.), solar cells, fuel cells, and the like.

The processor 32 can also be coupled to the GPS chipset 50, which can be configured to provide location information (e.g., longitude and latitude) regarding the current location of the device 30. It will be appreciated that the device 30 can acquire location information by way of any suitable location-determination method while remaining consistent with an example.

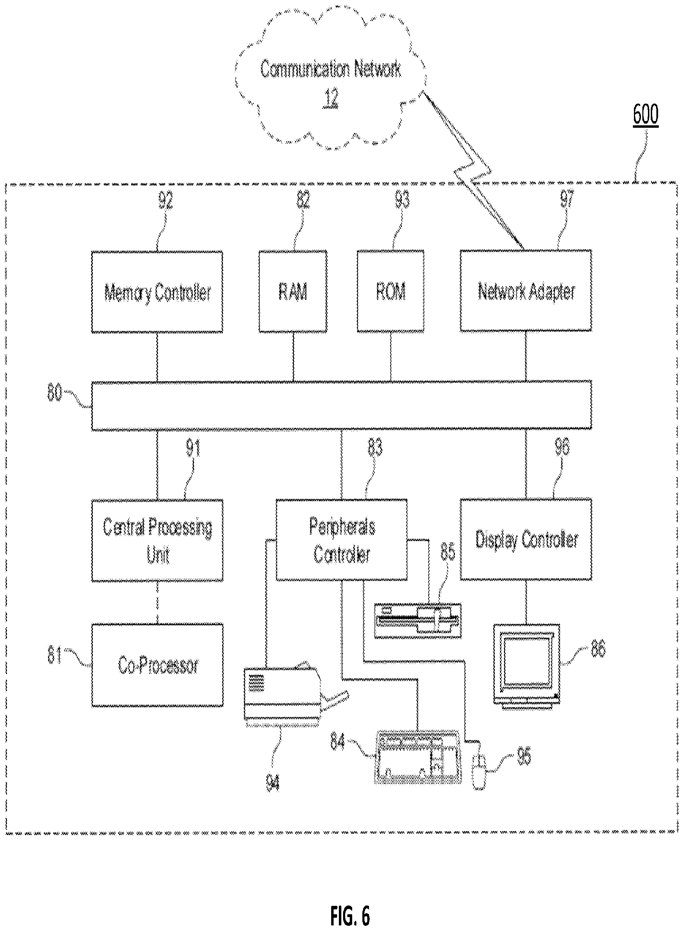

FIG. 6 is a block diagram of a computing system 600 which can also be used to implement components of the system or be part of the device 30. The computing system 600 can comprise a computer or server and can be controlled primarily by computer readable instructions, which can be in the form of software, wherever, or by whatever means such software is stored or accessed. Such computer readable instructions can be executed within a processor, such as central processing unit (CPU) 91, to cause computing system 600 to operate. In many workstations, servers, and personal computers, central processing unit 91 can be implemented by a single-chip CPU called a microprocessor. In other machines, the central processing unit 91 can comprise multiple processors. Coprocessor 81 can be an optional processor, distinct from main CPU 91, that performs additional functions or assists CPU 91.

In operation, CPU 91 fetches, decodes, and executes instructions, and transfers information to and from other resources via the computer's main data-transfer path, system bus 80. Such a system bus connects the components in computing system 600 and defines the medium for data exchange. System bus 80 typically includes data lines for sending data, address lines for sending addresses, and control lines for sending interrupts, and for operating the system bus. An example of such a system bus 80 is the Peripheral Component Interconnect (PCI) bus.

Memories coupled to system bus 80 include RAM 82 and ROM 93. Such memories can include circuitry that allows information to be stored and retrieved. ROMs 93 generally contain stored data that cannot easily be modified. Data stored in RAM 82 can be read or changed by CPU 91 or other hardware devices. Access to RAM 82 and/or ROM 93 can be controlled by memory controller 92. Memory controller 92 can provide an address translation function that translates virtual addresses into physical addresses as instructions are executed. Memory controller 92 can also provide a memory protection function that isolates processes within the system and isolates system processes from user processes. Thus, a program running in a first mode can access only memory mapped by its own process virtual address space; it cannot access memory within another process's virtual address space unless memory sharing between the processes has been set up.

In addition, computing system 600 can contain peripherals controller 83 responsible for communicating instructions from CPU 91 to peripherals, such as printer 94, keyboard 84, mouse 95, and disk drive 85.

Display 86, which is controlled by display controller 96, is used to display visual output generated by computing system 600. Such visual output can include text, graphics, animated graphics, and video. Display 86 can be implemented with a cathode-ray tube (CRT)-based video display, a liquid-crystal display (LCD)-based flat-panel display, gas plasma-based flat-panel display, or a touch-panel. Display controller 96 includes electronic components required to generate a video signal that is sent to display 86.

Further, computing system 600 can contain communication circuitry, such as for example a network adaptor 97, that can be used to connect computing system 500 to an external communications network, such as network 12 of FIG. 5, to enable the computing system 600 to communicate with other devices (e.g., device 30) of the network.

FIG. 7 illustrates a framework 700 employed by a software application (e.g., an algorithm) for evaluating captured images of rendered content and/or performing eye tracking. The framework 700 can be hosted remotely. Alternatively, the framework 700 can reside within the device 30 shown in FIG. 5 and/or be processed by the computing system 600 shown in FIG. 6. The machine learning model 710 is operably coupled to the stored training data 720 in a database.

In an example, the training data 720 can include attributes of thousands of objects. For example, the object can include eye positions and movements, pupil sizes, eye positions associated with various positions and the like. Attributes can include, but are not limited to the size, shape, orientation, position of the object, i.e., eye, gaze, etc. The training data 720 employed by the machine learning model 710 can be fixed or updated periodically. Alternatively, the training data 720 can be updated in real-time based upon the evaluations performed by the machine learning model 710 in a non-training mode. This is illustrated by the double-sided arrow connecting the machine learning model 710 and stored training data 720.

In operation, the machine learning model 710 may evaluate attributes of images/videos obtained by hardware (e.g., of the artificial reality system 100, HMD 110, etc.). The attributes of the captured image(s)/video(s) may be compared with respective attributes of stored training data 720 (e.g., prestored objects). The likelihood of similarity between each of the obtained attributes (e.g., of the captured image of an eye and the stored training data 720 (e.g., prestored objects) is given a confidence score. In one example, if the confidence score exceeds a predetermined threshold, the attribute is included in an image description that is ultimately communicated to the user via a user interface of a computing device (e.g., of the artificial reality system 100, HMD 110, etc.). In another example, the description can include a certain number of attributes which exceed a predetermined threshold to share with the user. The sensitivity of sharing more or less attributes can be customized based upon the needs of the particular user.

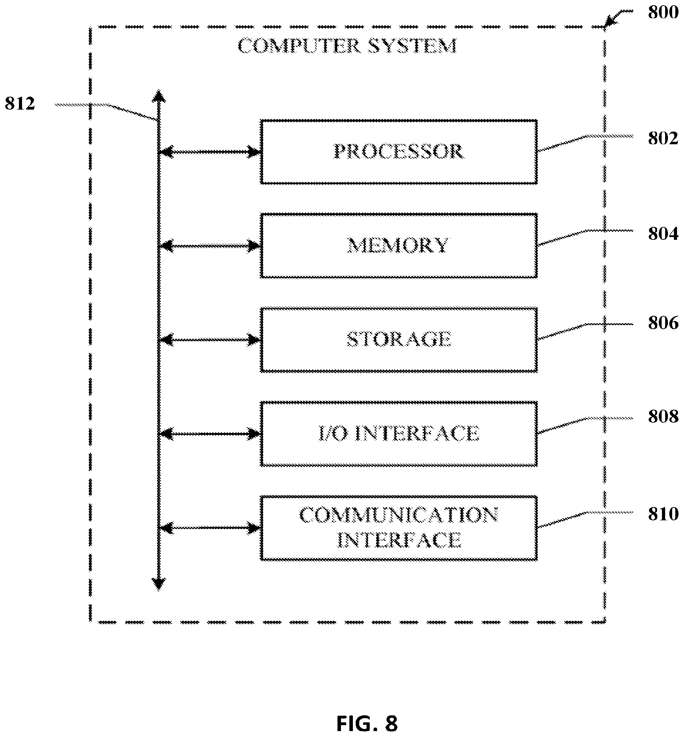

FIG. 8 illustrates an example computer system 800. In examples, one or more computer systems 800 perform one or more steps of one or more methods described or illustrated herein. In particular examples, one or more computer systems 800 provide functionality described or illustrated herein. In examples, software running on one or more computer systems 800 performs one or more steps of one or more methods described or illustrated herein or provides functionality described or illustrated herein. Examples may include one or more portions of one or more computer systems 800. Herein, a reference to a computer system can encompass a computing device, and vice versa, where appropriate. Moreover, a reference to a computer system can encompass one or more computer systems, where appropriate.

This disclosure contemplates any suitable number of computer systems 800. This disclosure contemplates computer system 800 taking any suitable physical form. As example and not by way of limitation, computer system 800 can be an embedded computer system, a system-on-chip (SOC), a single-board computer system (SBC) (such as, for example, a computer-on-module (COM) or system-on-module (SOM)), a desktop computer system, a laptop or notebook computer system, an interactive kiosk, a mainframe, a mesh of computer systems, a mobile telephone, a personal digital assistant (PDA), a server, a tablet computer system, or a combination of two or more of these. Where appropriate, computer system 800 can include one or more computer systems 800; be unitary or distributed; span multiple locations; span multiple machines; span multiple data centers; or reside in a cloud, which can include one or more cloud components in one or more networks. Where appropriate, one or more computer systems 800 can perform without substantial spatial or temporal limitations, one or more steps of one or more methods described or illustrated herein. As an example, and not by way of limitation, one or more computer systems 800 can perform in real time or in batch mode one or more steps of one or more methods described or illustrated herein. One or more computer systems 800 can perform at different times or at different locations one or more steps of one or more methods described or illustrated herein, where appropriate.

In example, computer system 800 includes a processor 802, memory 804, storage 806, an input/output (I/O) interface 808, a communication interface 810, and a bus 812. Although this disclosure describes and illustrates a particular computer system having a particular number of particular components in a particular arrangement, this disclosure contemplates any suitable computer system having any suitable number of any suitable components in any suitable arrangement.

In examples, processor 802 includes hardware for executing instructions, such as those making up a computer program. As an example and not by way of limitation, to execute instructions, processor 802 can retrieve (or fetch) the instructions from an internal register, an internal cache, memory 804, or storage 806; decode and execute them; and then write one or more results to an internal register, an internal cache, memory 804, or storage 806. In particular examples, processor 802 can include one or more internal caches for data, instructions, or addresses. This disclosure contemplates processor 802, including any suitable number of any suitable internal caches, where appropriate. As an example and not by way of limitation, processor 802 can include one or more instruction caches, one or more data caches, and one or more translation lookaside buffers (TLBs). Instructions in the instruction caches can be copies of instructions in memory 804 or storage 806, and the instruction caches can speed up retrieval of those instructions by processor 802. Data in the data caches can be copies of data in memory 804 or storage 806 for instructions executing at processor 802 to operate on; the results of previous instructions executed at processor 802 for access by subsequent instructions executing at processor 802 or for writing to memory 804 or storage 806; or other suitable data. The data caches can speed up read or write operations by processor 802. The TLBs can speed up virtual-address translation for processor 802. In particular examples, processor 802 can include one or more internal registers for data, instructions, or addresses. This disclosure contemplates processor 802 including any suitable number of any suitable internal registers, where appropriate. Where appropriate, processor 802 can include one or more arithmetic logic units (ALUs); be a multi-core processor; or include one or more processors 802. Although this disclosure describes and illustrates a particular processor, this disclosure contemplates any suitable processor.

In examples, memory 804 includes main memory for storing instructions for processor 702 to execute or data for processor 802 to operate on. As an example, and not by way of limitation, computer system 800 can load instructions from storage 806 or another source (such as, for example, another computer system 800) to memory 804. Processor 802 can then load the instructions from memory 804 to an internal register or internal cache. To execute the instructions, processor 802 can retrieve the instructions from the internal register or internal cache and decode them. During or after execution of the instructions, processor 802 can write one or more results (which can be intermediate or final results) to the internal register or internal cache. Processor 802 can then write one or more of those results to memory 804. In particular examples, processor 802 executes only instructions in one or more internal registers or internal caches or in memory 804 (as opposed to storage 806 or elsewhere) and operates only on data in one or more internal registers or internal caches or in memory 804 (as opposed to storage 806 or elsewhere). One or more memory buses (which can each include an address bus and a data bus) can couple processor 802 to memory 804. Bus 812 can include one or more memory buses, as described below. In examples, one or more memory management units (MMUs) reside between processor 802 and memory 804 and facilitate accesses to memory 804 requested by processor 802. In particular examples, memory 804 includes random access memory (RAM). This RAM can be volatile memory, where appropriate. Where appropriate, this RAM can be dynamic RAM (DRAM) or static RAM (SRAM). Moreover, where appropriate, this RAM can be single-ported or multi-ported RAM. This disclosure contemplates any suitable RAM. Memory 804 can include one or more memories 804, where appropriate. Although this disclosure describes and illustrates particular memory, this disclosure contemplates any suitable memory.

In examples, storage 806 includes mass storage for data or instructions. As an example, and not by way of limitation, storage 806 can include a hard disk drive (HDD), a floppy disk drive, flash memory, an optical disc, a magneto-optical disc, magnetic tape, or a Universal Serial Bus (USB) drive, or a combination of two or more of these. Storage 806 can include removable or non-removable (or fixed) media, where appropriate. Storage 806 can be internal or external to computer system 800, where appropriate. In examples, storage 806 is non-volatile, solid-state memory. In particular examples, storage 806 includes read-only memory (ROM). Where appropriate, this ROM can be mask-programmed ROM, programmable ROM (PROM), erasable PROM (EPROM), electrically erasable PROM (EEPROM), electrically alterable ROM (EAROM), or flash memory or a combination of two or more of these. This disclosure contemplates mass storage 806 taking any suitable physical form. Storage 806 can include one or more storage control units facilitating communication between processor 802 and storage 806, where appropriate. Where appropriate, storage 806 can include one or more storages 806. Although this disclosure describes and illustrates particular storage, this disclosure contemplates any suitable storage.

In examples, I/O interface 808 includes hardware, software, or both, providing one or more interfaces for communication between computer system 800 and one or more I/O devices. Computer system 800 can include one or more of these I/O devices, where appropriate. One or more of these I/O devices can enable communication between a person and computer system 800. As an example and not by way of limitation, an I/O device can include a keyboard, keypad, microphone, monitor, mouse, printer, scanner, speaker, still camera, stylus, tablet, touch screen, trackball, video camera, another suitable I/O device or a combination of two or more of these. An I/O device can include one or more sensors. This disclosure contemplates any suitable I/O devices and any suitable I/O interfaces 808 for them. Where appropriate, I/O interface 808 can include one or more device or software drivers, enabling processor 802 to drive one or more of these I/O devices. I/O interface 808 can include one, or more I/O interfaces 808, where appropriate. Although this disclosure describes and illustrates a particular I/O interface, this disclosure contemplates any suitable I/O interface.

In examples, communication interface 810 includes hardware, software, or both providing one or more interfaces for communication (such as, for example, packet-based communication) between computer system 800 and one or more other computer systems 800 or one or more networks. As an example and not by way of limitation, communication interface 810 can include a network interface controller (NIC) or network adapter for communicating with an Ethernet or other wire-based network or a wireless NIC (WNIC) or wireless adapter for communicating with a wireless network, such as a WI-FI network. This disclosure contemplates any suitable network and any suitable communication interface 810 for it. As an example, and not by way of limitation, computer system 800 can communicate with an ad hoc network, a personal area network (PAN), a local area network (LAN), a wide area network (WAN), a metropolitan area network (MAN), or one or more portions of the Internet, or a combination of two or more of these. One or more portions of one or more of these networks can be wired or wireless. As an example, computer system 800 can communicate with a wireless PAN (WPAN) (such as, for example, a BLUETOOTH WPAN), a WI-FI network, a WI-MAX network, a cellular telephone network (such as, for example, a Global System for Mobile Communications (GSM) network), or other suitable wireless network or a combination of two or more of these. Computer system 800 can include any suitable communication interface 810 for any of these networks, where appropriate. Communication interface 810 can include one or more communication interfaces 810, where appropriate. Although this disclosure describes and illustrates a particular communication interface, this disclosure contemplates any suitable communication interface.

In particular examples, bus 812 includes hardware, software, or both coupling components of computer system 800 to each other. As an example and not by way of limitation, bus 812 can include an Accelerated Graphics Port (AGP) or other graphics bus, an Enhanced Industry Standard Architecture (EISA) bus, a front-side bus (FSB), a HYPERTRANSPORT (HT) interconnect, an Industry Standard Architecture (ISA) bus, an INFINIBAND interconnect, a low-pin-count (LPC) bus, a memory bus, a Micro Channel Architecture (MCA) bus, a Peripheral Component Interconnect (PCI) bus, a PCI-Express (PCIe) bus, a serial advanced technology attachment (SATA) bus, a Video Electronics Standards Association local (VLB) bus, or another suitable bus or a combination of two or more of these. Bus 812 can include one or more buses 812, where appropriate. Although this disclosure describes and illustrates a particular bus, this disclosure contemplates any suitable bus or interconnect.

Herein, a computer-readable non-transitory storage medium or media can include one or more semiconductor-based or other integrated circuits (ICs) (such, as for example, field-programmable gate arrays (FPGAs) or application-specific ICs (ASICs)), hard disk drives (HDDs), hybrid hard drives (HHDs), optical discs, optical disc drives (ODDs), magneto-optical discs, magneto-optical drives, floppy diskettes, floppy disk drives (FDDs), magnetic tapes, solid-state drives (SSDs), RAM-drives, SECURE DIGITAL cards or drives, any other suitable computer-readable non-transitory storage media, computer readable medium or any suitable combination of two or more of these, where appropriate. A computer-readable non-transitory storage medium can be volatile, non-volatile, or a combination of volatile and non-volatile, where appropriate.

Herein, "or" is inclusive and not exclusive, unless expressly indicated otherwise or indicated otherwise by context. Therefore, herein, "A or B" means "A, B, or both," unless expressly indicated otherwise or indicated otherwise by context. Moreover, "and" is both joint and several, unless expressly indicated otherwise or indicated otherwise by context. Therefore, herein, "A and B" means "A and B, jointly or severally," unless expressly indicated otherwise or indicated otherwise by context.

The scope of this disclosure encompasses all changes, substitutions, variations, alterations, and modifications to the examples described or illustrated herein that a person having ordinary skill in the art would comprehend. The scope of this disclosure is not limited to the examples described or illustrated herein. Moreover, although this disclosure describes and illustrates respective examples herein as including particular components, elements, feature, functions, operations, or steps, any of these examples can include any combination or permutation of any of the components, elements, features, functions, operations, or steps described or illustrated anywhere herein that a person having ordinary skill in the art would comprehend. Furthermore, reference in the appended claims to an apparatus or system or a component of an apparatus or system being adapted to, arranged to, capable of, configured to, enabled to, operable to, or operative to perform a particular function encompasses that apparatus, system, component, whether or not it or that particular function is activated, turned on, or unlocked, as long as that apparatus, system, or component is so adapted, arranged, capable, configured, enabled, operable, or operative. Additionally, although this disclosure describes or illustrates particular examples as providing particular advantages, particular examples can provide none, some, or all of these advantages.

The foregoing description of the examples has been presented for the purpose of illustration; it is not intended to be exhaustive or to limit the patent rights to the precise forms disclosed. Persons skilled in the relevant art can appreciate that many modifications and variations are possible in light of the above disclosure.

Some portions of this description describe the embodiments and examples in terms of applications and symbolic representations of operations on information. These application descriptions and representations are commonly used by those skilled in the data processing arts to convey the substance of their work effectively to others skilled in the art. These operations, while described functionally, computationally, or logically, are understood to be implemented by computer programs or equivalent electrical circuits, microcode, or the like. Furthermore, it has also proven convenient at times, to refer to these arrangements of operations as modules, without loss of generality. The described operations and their associated modules may be embodied in software, firmware, hardware, or any combinations thereof.

Any of the steps, operations, or processes described herein may be performed or implemented with one or more hardware or software modules, alone or in combination with other devices. In one example, a software module is implemented with a computer program product comprising a computer-readable medium containing computer program code, which can be executed by a computer processor for performing any or all of the steps, operations, or processes described.

Examples also may relate to an apparatus for performing the operations herein. This apparatus may be specially constructed for the required purposes, and/or it may comprise a computing device selectively activated or reconfigured by a computer program stored in the computer. Such a computer program may be stored in a non-transitory, tangible computer readable storage medium, or any type of media suitable for storing electronic instructions, which may be coupled to a computer system bus. Furthermore, any computing systems referred to in the specification may include a single processor or may be architectures employing multiple processor designs for increased computing capability.

Examples also may relate to a product that is produced by a computing process described herein. Such a product may comprise information resulting from a computing process, where the information is stored on a non-transitory, tangible computer readable storage medium and may include any embodiment or example of a computer program product or other data combination described herein.

Finally, the language used in the specification has been principally selected for readability and instructional purposes, and it may not have been selected to delineate or circumscribe the inventive subject matter. It is therefore intended that the scope of the patent rights be limited not by this detailed description, but rather by any claims that issue on an application based hereon. Accordingly, the disclosure of the examples is intended to be illustrative, but not limiting, of the scope of the patent rights, which is set forth in the following claims.

## Claims

1. An apparatus comprising:
one or more processors;
at least one memory storing instructions;
at least one digital micro-mirror device configured to:
emit light, comprising one or more binary images or one or more grayscale images, onto at least one eye of a user; and
a plurality of single pixel detectors configured to:
determine a reflection of the light from the at least one eye;
determine, by a first single pixel detector of the plurality of single pixel detectors, a first signal associated with the reflection of the light from a retina of the at least one eye;
determine, by a second single pixel detector of the plurality of single pixel detectors, a second signal associated with the reflection of the light from the retina of the at least one eye; and
wherein when the one or more processors execute the instructions, the apparatus is configured to:
perform a transformation on the first signal and the second signal to determine at least one grayscale image of the retina; and
determine at least one three-dimensional (3D) shape of the retina based on performing at least one 3D reconstruction associated with one or more pixels of the grayscale image of the retina.

2. The apparatus of claim 1, wherein when the one or more processors further execute the instructions, the apparatus is configured to:
track one or more movements of the at least one eye based in part on the determined at least one 3D shape of the retina;
preferably wherein when the one or more processors further execute the instructions, the apparatus is configured to:
perform the track the one or more movements of the at least one eye based in part on determining one or more eye tracking parameters associated with the 3D shape of the retina;
further preferably wherein the one or more eye tracking parameters comprise at least one of an angle of eye-gaze of the at least one eye, a torsion of the at least one eye, a shape of the at least one eye, a current focal distance of the at least one eye, a dilation of a pupil of the at least one eye, an inter-pupillary distance of the at least one eye and a second eye of the user, a translation of the at least one eye, an accommodation of the at least one eye, other features of a state of the at least one eye, or a combination thereof.

3. The apparatus of claim 1 or 2, wherein prior to the performing the transformation, the at least one digital micro-mirror device is further configured to:
receive the first signal and the second signal from the plurality of single pixel detectors.

4. The apparatus of any preceding claim, wherein the at least one digital micro-mirror device is further configured to:
render content to the at least one eye based on a determined location of a pupil of the at least one eye.

5. The apparatus of claim 4, wherein when the one or more processors further execute the instructions, the apparatus is further configured to perform the determined location of the pupil;
preferably further comprising:
a spatial light modulator;
wherein the light is spatially modulated, by the at least one digital micro-mirror device or by the spatial light modulator, based on the determined location of the pupil.

6. The apparatus of any preceding claim, wherein when the one or more processors further execute the instructions, the apparatus is configured to:
determine at least one characteristic of the at least one eye based on the at least one grayscale image of the retina.

7. A method comprising:
emitting by at least one digital micro-mirror device, light, comprising one or more binary images or one or more grayscale images, onto at least one eye of a user;
determining, by a plurality of single pixel detectors, a reflection of the light from the at least one eye;
determining, by a first single pixel detector of the plurality of single pixel detectors, a first signal associated with the reflection of the light from a retina of the at least one eye;
determining, by a second single pixel detector of the plurality of single pixel detectors, a second signal associated with the reflection of the light from the retina of the at least one eye; performing, by the at least one digital micro-mirror device, a transformation on the first signal and the second signal to determine at least one grayscale image of the retina; and
determining, by the at least one digital micro-mirror device, at least one three-dimensional (3D) shape of the retina based on performing at least one 3D reconstruction associated with one or more pixels of the grayscale image of the retina.

8. The method of claim 7, further comprising:
tracking one or more movements of the at least one eye based in part on the determined at least one 3D shape of the retina;
preferably further comprising:
performing the tracking the one or more movements of the at least one eye based in part on determining one or more eye tracking parameters associated with the 3D shape of the retina;
further preferably wherein the one or more eye tracking parameters comprise at least one of an angle of eye-gaze of the at least one eye, a torsion of the at least one eye, a shape of the at least one eye, a current focal distance of the at least one eye, a dilation of a pupil of the at least one eye, an inter-pupillary distance of the at least one eye and a second eye of the user, a translation of the at least one eye, an accommodation of the at least one eye, other features of a state of the at least one eye, or a combination thereof.

9. The method of claim 7 or 8, wherein prior to the performing the transformation, the method further comprises:
receiving, by the least one digital micro-mirror device, the first signal and the second signal from the plurality of single pixel detectors.

10. The method of any of claims 7 to 9, further comprising:
rendering, by the at least one digital micro-mirror device, content to the at least one eye based on a determined location of a pupil of the at least one eye.

11. The method of claim 10, further comprising:
performing, by at least one of the plurality of single pixel detectors, the determined location of the pupil.

12. The method of claim 10 or 11, further comprising:
spatially modulating the light, by the at least one digital micro-mirror device or by a spatial light modulator, based on the determined location of the pupil.

13. The method of any of claims 7 to 12, further comprising:
determining at least one characteristic of the at least one eye based on the at least one grayscale image of the retina.

14. A computer-readable storage medium storing computer executable instructions that, when executed by a computer, cause the computer to carry out the method of any of claims 7 to 13.

15. A computer program product comprising instructions which, when the program is executed by a computer, cause the computer to carry out the method of any of claims 7 to 13.
